(19) Europäisches Patentamt
European Patent Office
Office européen des brevets

(11) Publication number : 0 589 600 A2

# (12) EUROPEAN PATENT APPLICATION

(21) Application number : 93307198.7

(22) Date of filing : 13.09.93

(51) Int. Cl.$^5$ : C12Q 1/68, G01N 5/00

(30) Priority : 14.09.92 JP 270981/92
31.08.93 JP 237203/93
31.08.93 JP 237204/93

(43) Date of publication of application :
30.03.94 Bulletin 94/13

(84) Designated Contracting States :
CH DE FR GB IT LI NL SE

(71) Applicant : SOGO PHARMACEUTICAL COMPANY LIMITED
6-2 Otemachi 2-chome
Chiyoda-ku Tokyo (JP)

(72) Inventor : Okahata, Yoshio
2-11, Nijigaoka 1-chome, Asou-ku
Kawasaki-shi, Kanagawa-ken (JP)
Inventor : Toshinori, Sato
RB-12, 24 Fujigaoka 3-chome
Fujisawa-shi, Kanagawa-ken (JP)
Inventor : Kuniharu, Ijiro, No. 503, Kahsa Saginumamm
3-2 Tsuchihashi 3-chome, Miyamae-ku
Kawasaki-shi, Kanagawa-ken (JP)

(74) Representative : Linn, Samuel Jonathan et al
MEWBURN ELLIS 2 Cursitor Street
London EC4A 1BQ (GB)

## (54) Method of detecting biochemical substances and sensor for use in said method.

(57) A method of detecting biochemical substances which method comprises chemically binding and immobilizing a probe DNA having a sequence complementary with a sequence specific to a DNA of a biochemical substance onto an electrode of a frequency conversion element, hybridizing the probe DNA with a sample to detect said DNA as a target DNA having a sequence specific to the probe DNA, and measuring frequency changes corresponding to mass changes due to hybridization to detect and identify a hybridized DNA ; and a sensor use in the above method.

EP 0 589 600 A2

The present invention relates to a method of detecting biochemical substances and sensor for use in said method.

Japanese Patent Application Laid-Open No. 210198/91 discloses analysis separation and determination in the DNA hybridization reaction according to a process which comprises carrying out DNA hybridization reaction followed by separating by means of electrophoresis, or followed by labelling with radioisotope for determination. However, the above processes show such disadvantages that a limited space is required for labelling, that a special technique is required for handling radioisotopes and that it takes a long time for procedures, treatments and determination during labelling, etc.

Japanese Patent Application Laid-Open No. 210198/92 discloses a method of detecting a product obtained by an enzymatic reaction by hybridizing an intended DNA with a DNA modified by an antigen having a complementary sequence, followed by reacting with an enzyme-modified antibody. However, the above process has such disadvantages that quantitative evaluation is poor due to deactivation of enzymatic activity, etc. and that it takes a long time for procedures, treatments and measurements during the preparation of the enzyme-modified antibody, etc.

Investigation and determination of DNA cleavage in the DNA catalytic hydrolysis in the presence of DNA DNase as well as determination of binding constant have been made for analyzing phenomena particular to DNA, for example, hybridization of DNAs of biochemical substances, intercalation of biochemical substances into DNAs, etc., by the spectroscopic techniques such as measurements of changes in absorbance of ultraviolet spectrum, measurements of circular dichroism spectrum, etc., resulting in raising such problems that apparatuses are expensive and that quantitative evaluation is poor, and so forth.

It is an object of the present invention to provide a method of detecting biochemical substances, which is capable of simply and directly analyzing DNA hybridization reaction, and separating and determining hybridized DNA, and capable of accurately, quantitatively and simply hybridizing DNA to detect biochemical substances in an aqueous phase or gaseous phase, without the above disadvantages in the prior art, for example, requirements of special techniques, poor quantitative evaluation, taking a long time for measurements; and to provide a sensor used in the above method.

It is another object of the present invention to provide a method of detecting biochemical substances, which is capable of simply and directly analyzing DNA hybridization reaction, determining binding constants on DNA hybridization to conduct kinetic analysis and separating and determining hybridized DNA, and capable of accurately, quantitatively and simply hybridizing DNA to detect biochemical substances, without the above disadvantages in the prior art, for example, requirements of special technique, poor quantitative evaluation, taking a long time for measurements, use of expensive apparatus, etc.; and to provide a sensor used in the above method.

A first embodiment of the present invention provides a method of detecting biochemical substances which method comprises chemically binding and immobilizing a probe DNA having a sequence complementary with a sequence specific to a DNA of a biochemical substance onto an electrode of a frequency conversion element, hybridizing the probe DNA with a sample to detect said DNA as a target DNA having a sequence specific to the probe DNA, and measuring frequency changes corresponding to mass changes due to hybridization to detect and identify a hybridized DNA.

A second embodiment of the present invention provides a method of detecting biochemical substances which method comprises chemically binding and immobilizing a DNA constituting a biochemical substance and specifically adsorbing a protein as a biochemical substance onto an electrode of a freqency convension element, adsorbing a sample to detect the protein, and measuring frequency changes corresponding to mass changes due to adsorption onto the electrode to detect the protein.

A third embodiment of the present invention provides a sensor used in the method of the first embodiment of the present invention, which sensor comprises a frequency conversion element and a probe DNA chemically bound and immobilized onto an electrode of the frequency conversion element and having a sequence complementary with a sequence specific to a DNA of a biochemical substance.

A fourth embodiment of the present invention provides a sensor used in the method of the second embodiment of the present invention, which sensor comprises a frequency conversion element and a DNA constituting a biochemical substance, specifically adsorbing a protein as a biochemical substance and being chemically bound and immobilized onto an electrode of the frequency conversion element.

## Brief Description of the Drawings:

Fig. 1 is a schematic view of an example of a double-stranded DNA with a thiol group in the present invention.

Fig. 2 is a graph for explaining an example of the method of immobilizing the probe DNA onto the electrode

in the present invention.

Fig. 3 is a graph showing an example of DNA cleavage changes with time in the present invention.

Fig. 4 is a graph showing a relationship between dipping time and dry weight of probe DNA for explaining an example of preferable embodiments of the present invention.

Fig. 5 is a graph showing a time course of binding of complementary M13 DNA target with probe DNA for explaining an example of preferable embodiments of the present invention.

Fig. 6 is a graph showing a time course of binding of non-complementary M13 DNA with probe DNA for explaining an example of preferable embodiments of the present invention.

Fig. 7 is a graph showing a relationship between temperature and frequency changes for explaining an example of preferable embodiments of the present invention.

Fig. 8 is a graph showing a relationship between temperature and calibrated frequency changes for explaining an example of preferable embodiments of the present invention.

Fig. 9 is a graph showing a relationship between temperature and absorbance for explaning an example of preferable embodiments of the present invention.

Fig. 10 is a graph showing a time course of frequency changes for explaining an example of preferable embodiments of the present invention.

Fig. 11 is a graph showing a time course of frequency changes for explaining an example of preferable embodiments of the present invention.

Fig. 12 is a graph showing a time course of frequency changes for explaining an example of preferable embodiments of the present invention.

Fig. 13 is a graph showing a relationship between temperature and frequency changes for explaining an example of preferable embodiments of the present invention.

Fig. 14 is a graph showing a time course of frequency changes depending on different amounts of immobilized DNA respectively for explaining an example of preferable embodiments of the present invention.

Fig. 15 is a graph showing a relationship of an immobilized amount of probe DNA with a binding amount of complementary M13 DNA or with a percentage of a bound amount of complementary M13 DNA to an added amount of complementary M13 DNA for explaining an example of preferable embodiments of the present invention.

Fig. 16 is a graph showing a relationship between concentration of complementary M13 DNA and bound amount thereof for explaining an example of preferable embodiments of the present invention.

Fig. 17 is a graph showing a relationship between concentration of 10-mer DNA and bound amount thereof for explaining an example of preferable embodiments of the present invention.

Fig. 18 is a graph showing frequency changes with time for explaining an example of preferable embodiments of the present invention.

Fig. 19 is a graph showing a relationship between concentrations of target DNA and saturated adsorbed amounts corresponding to respective concentrations thereof for explaining an example of preferable embodiments of the present invention.

Fig. 20 is a graph showing a relationship between concentrations of target DNA and ratios of concentrations of target DNA to $\Delta$ m for explaining an example of preferable embodiments of the present invention.

The biochemical substances to be detected by the method and sensor according to the present invention may include ones having a specific sequence and ones having specific bases constituting the sequence, and more specifically may include nucleic acids constituting living things, particularly microorganisms such as various Funji and virus and having a specific sequence, bases constituting the nucleic acid and nucleic acid components containing the base.

Examples of the above nucleic acid may include DNA, RNA and the like.

Examples of bases constituting the above nucleic acid and the nucleic acid components containing the base may include bases such as adenine, guanine, cytosine, thymine, uracil and the like; nucleosides such as cytidine, uridine, deoxyadenosine, deoxyguanosine, adenosine, guanosine, deoxythymidine, deoxycytidine, and the like; nucleotides such as adenosine monophosphate, adenosine diphosphate, adenosine triphosphate, deoxycytidine monophosphate, deoxyguanosine triphosphate, deoxythymidine monophosphate uridine monophosphate, and the like; and the like. Of these, DNA and RNA are preferred.

The above DNA may include single-stranded DNAs, double-stranded DNAs forming a double helical structure, and triple-stranded DNAs forming a triple helical structure.

Examples of the protein specifically adsorbed onto a DNA of a biochemical substance to be detected according to the present invention may include lac. repressor protein known as a DNA herical unstabilized protein, a T antigen produced from a monkey virus, and the like.

The frequency conversion element used in the present invention may include a crystal oscillator, a surface acostic wave device, and the like.

The probe DNA used in the present invention is composed of a sequence complementary with a sequence specific to a DNA of the above biochemical substance and may be obtained by use of a DNA synthesizer according to the phosphoramidite method. The probe DNA may include single-stranded DNAs and double-stranded DNAs.

The probe DNA may also be chemically bound and immobilized onto the electrode in the form of a complex with a cationic lipid represented by the following general formula (I):

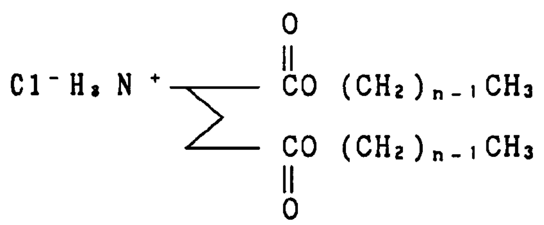

... (I)

where n is an integer of 4, 6 or 8 (hereinafter may be simply referred to as $2C_4N^+$, $2C_6N^+$ and $2C_8N^+$ respectively). The above complex may be referred to as a DNA-lipid complex. The DNA-lipid complex may be obtained by mixing both DNA and lipid.

The use of the DNA-lipid complex results in controlling hydrolysis by DNase, because the phosphate group of the DNA is protected, and in improving interaction with a lipid film, because the surface of the DNA is hydrophobilized.

The target DNA used in the present invention includes ones constituting DNAs of the above biochemical substances and having a sequence specific to the above probe DNA, and has a specific sequence complementary with the sequence of the probe DNA. The target DNA may include single-stranded DNAs and double-stranded DNAs.

Examples of the method of chemically binding and immobilizing the probe DNA onto the electrode of the frequency conversion element in the present invention may include (I) a process which comprises introducing a thiol group onto an end of the complementary sequence of the probe DNA, dipping a crystal oscillator having a gold electrode as the frequency conversion element into an aqueous solution of the probe DNA with the thiol group to be reacted and chemically bound, followed by removing the frequency conversion element and drying; (2) a process which comprises introducing a thiol group onto an end of the complementary sequence of the probe DNA, dropping an aqueous solution of the probe DNA with the thiol group onto a crystal oscillator having a gold electrode as the frequency conversion element in a gaseous phase to be reacted and chemically bound, followed by washing with a deionized water or with an aqueous solution not containing any DNA to be hybridized with the probe DNA, and by drying; and the like. Examaples of the above thiol group may include s-trityl-3-mercaptopropyloxy- $\beta$ -cyanoethyl-N,N-diisopropylaminophosphoramidite, and the like. Introduction of the above thiol group onto the end of the complementary sequence of the probe DNA may be carried out by the phosphoramidite method.

The amount of the probe DNA to be chemically bound and immobilized onto the electrode of the frequency conversion element in the present invention may vary depending on a size of the target DNA to be hybridized therewith, but is normally in the range of 1 to 1500 ng, preferably 100 to 200 ng.

In a preferable embodiment of the first embodiment of the present invention, an aqueous sample to detect said DNA is dropped onto the frequency conversion element with the immobilized probe DNA in a gaseous phase to be hybridized.

In a preferable embodiment of the first embodiment of the present invention, the frequency conversion element with the immobilized probe DNA is dipped into water, followed by injecting the sample to detect the DNA into the water to be hybridized.

In a preferable embodiment of the first embodiment of the present invention, the probe DNA is a single-stranded DNA and the frequency conversion element with the immobilized DNA is dipped into water, follwed by injecting into the water a sample DNA molecule selected from a group consisting of a DAN molecule having substantially the same number of bases or less, substantially the same molecular weight or less and at least one base pair specific to the probe DNA, a DNA molecule having substantially the same number of bases or less, substantially the same molecular weight or less and having no base pairs specific to the probe DNA and mixtures thereof, as a target DNA molecule to be hybridized with the probe DNA, and measuring frequency changes corresponding to mass changes due to hybridization to detect and identify a hybridized DNA molecule.

In the above preferable embodiment of the first embodiment of the present invention, the DNA molecule having at least one base pair specific to the probe DNA, the DNA molecule having no base pairs specific to

the probe DNA and mixtures thereof are subjected to hybridization reaction respectively, and frequency changes corresponding to mass changes due to hybridization are measured respectively resulting in making it possible to detect and identify hybridized DNA molecule thus formed.

In the above preferable embodiment of the first embodiment of the present invention, since the molecular weight of the target DNA molecule is much smaller than those of the target DNA, it is made possible to clearly distinguish frequency changes in the case where the sequence of the target DNA molecule is complementary with that of the probe DNA from frequency changes in the case where the sequence of the target DNA molecule is not complementary with that of the probe DNA without needing to washing the frequency conversion element with the immobilized probe DNA with deionized water or with an aqueous solution not containing the target DNA after frequency changes have reached a saturated state, or without needing to carry out measurement of frequency changes under such temperature conditions that non-specific adsorption onto the frequency conversion element with the immobilized probe DNA is controlled, resulting in making it possible to analyze more in detail complementarity with the sequence of the probe DNA.

In a preferable embodiment of the first embodiment of the present invention, the probe DNA is a single-stranded DNA and the frequency conversion element with the immobilized probe DNA is dipped into water, followed by injecting into the water a sample DNA molecule comprising substantially the same number of bases as the probe DNA or less or comprising a nucleic acid component containing the above bases as a target DNA molecule to be hybridized with the probe DNA, and measuring frequency changes corresponding to mass changes due to hybridization to detect and identify a hybridized DNA molecule.

In a preferable embodiment of the first embodiment of the present invention, the frequency conversion element with the immobilized probe DNA is dipped into water, followed by injecting a sample to detect the DNA into the water, hybridizing the probe DNA with the sample as a target DNA having a sequence specific to the probe DNA, and measuring frequency changes with time to determine a binding constant (K) on hybridization.

In a preferable embodiment of the first embodiment of the present invention, the binding constant (K) on hybridization may be determined by a method which comprises measuring frequency changes with time on hybridization in water by use of a crystal oscillator, removing the crystal oscillator out of the water at a time when an adsorption equilibrium has been reached, separately dipping the crystal oscillator into a large amount of water, and further measuring frequency changes with time to determine a binding rate constant ($k_1$) from an initial frequency-decreasing rate or from an initial increasing rate ($V_1$) of an adsorbed amount, to determine a dessociation rate constant ($k_2$) from an initial frequency-increasing rate or from an initial decreasing rate ($V_2$) of an adsorbed amount, and to determine a binding constant (K) according to the following equations (1) to (6):

$$[\text{probe}] + [\text{target}] \parallel [\text{ds - DNA}] \quad (1)$$
$$V_1 = k_1 [\text{probe}][\text{target}] \quad (2)$$
$$k_1 = V_1/[\text{probe}][\text{target}] \quad (3)$$
$$V_2 = k_2[\text{ds - DNA}] \quad (4)$$
$$k_2 = V_2/[\text{ds - DNA}] \quad (5)$$
$$K = k_1/k_2 \quad (6)$$

where [probe]: probe concentration (mol/ℓ);
[target]: target concentration (mol/ℓ );
[ds-DNA]: concentration of hybridized double-stranded DNA (mol/ ℓ); $V_1$: initial adsorption rate (mol/s); $k_1$: binding rate constant ($M^{-1}S^{-1}$);
$V_2$: initial dessociation rate (mol/s); $k_2$: dessociation rate constant ($s^{-1}$); and K: binding constant ($M^{-1}$).

In a preferable embodiment of the first embodiment of the present invention, the binding constant (K) on hybridization may also be determined by a method which comprises dipping a frequency conversion element with immobiolized probe DNA into water, injecting a predetermined amount of target DNA into the water to be hybridized, repeating the procedures to determine an adsorbed amount of the target DNA when reached adsorption equilibrium with varied injecting amounts, determining a correlation between an initial concentration of target DNA, i.e. $[\text{target}]_0$ and a saturated adsorbed amount ($\Delta$ m), where $[\text{target}]_0$ is a concentration of target DNA at a time when the target DNA is injected, the saturated adsorbed amount ($\Delta$ m) corresponding to respective $[\text{target}]_0$ means an adsorbed amount of the target DNA when adsorption equilibrium is reached respectively, applying the following equation (I):

$$[\text{target}]_0/\Delta m = (1/\Delta m_{max})[\text{target}] + (1/\Delta m_{max}K) \quad (I)$$

where $[\text{target}]_0$, $\Delta$ m and K are as defined above, and $\Delta m_{max}$ means a saturated adsorbed amount of target DNA onto the probe DNA, and plotting a correlation between $[\text{target}]_0$ and $[\text{target}]_0/\Delta$ m taking $[\text{target}]_0$ as a X axis and $[\text{target}]_0/\Delta$ m as Y axis to determine the saturated adsorbed amount ($\Delta m_{max}$) of target DNA onto the probe DNA from a reciprocal number of a slope of a straight line through the above plots and to determine a binding constant (K) from a value of a Y axis intercept thus obtained.

In a preferable embodiment of the first embodiment of the present invention, the electrode is a gold electrode and the probe DNA is single-stranded and immobilized onto the gold electrode by a process which comprises introducing a thiol group onto an end of the sequence of the probe DNA, hybridizing the probe DNA having the thiol group with a DNA having a complementary sequence to form a double-stranded DNA, dipping the frequency conversion element into an aqueous solution of the double-stranded DNA with thiol group to be chemically bound, heating at a melting temperature or higher of the double-stranded DNA to desorb a single-stranded DNA not having been chemically bound on the electrode, followed by removing the frequency conversion element, and drying.

Immobilization by the above process makes it possible to uniformly immobilize the probe DNA onto the electrode and to improve the efficiency of hybridization with the target DNA.

In a preferable embodiment of the first embodiment of the present invention, the electrode is a gold electrode and the probe DNA is single-stranded and immobilized onto the gold electrode by a process which comprises introducing a thiol group onto an end of the sequence of the probe DNA, hybridizing the probe DNA having the thiol group with a DNA having a complementary sequence to form a double-stranded DNA, dropping an aqueous solution of the double-stranded DNA with thiol group onto the frequency conversion element in a gaseous phase to be reacted and chemically bound, heating at a melting temperature or higher of the double-stranded DNA to desorb a single-stranded DNA not having been chemically bound on the electrode, followed by washing with a deionized water or with an aqueous solution not containing any DNA to be hybridized with the probe DNA, and by drying.

Immobilization by the above process makes it possible to uniformly immobilize the probe DNA onto the electrode and to improve the efficiency of hybridization with the target DNA.

In a preferable embodiment of the first embodiment of the present invention, the DNA molecule having an sequence specific to the probe DNA is obtained by a process which comprises dipping the frequency conversion element with the immobilized probe DNA into an aqueous solution containing respective monomer nucleotides constituting the DNA molecule, arranging respective monomer nucleotides so as to form complementary base pairs with the probe DNA, and polymerizing the arranged monomer nucleotides in the presence of an enzyme such as polymerase, obtained by cutting off the complementary sequence of a biochemical substance having the same, or by a chemical synthesis by use of a DNA synthesizer.

In a preferable embodiment of the first embodiment of the present invention, the DNA is double-stranded, the probe DNA is a single-stranded DNA constituting the double-stranded DNA, and the target DNA is another single-stranded DNA constituting the double-stranded DNA and complementary with the probe DNA.

In a preferable embodiment of the first embodiment of the present invention, the DNA is triple-stranded, the probe DNA is a double-stranded DNA constituting the triple-stranded DNA, and the target DNA is another single-stranded DNA constituting the triple-stranded DNA and complementary with the probe DNA.

In a preferable embodiment of the first embodiment of the present invention, the DNA is triple-stranded the probe DNA is a single-stranded DNA constituting the triple-stranded DNA, and the target DNA is other double-stranded DNA constituting the triple-stranded DNA and complementary with the probe DNA.

In a preferable embodiment of the first embodiment of the present invention, the probe DNA is immobilized in the form of a complex with a cationic lipid.

In a preferable embodiment of the present invention, measurement of frequency changes is carried out in gaseous phase by a process which comprises hybridizing, followed by washing with deionized water or with an aqueous solution not containing the target DNA, and by drying.

In a preferable embodiment of the present invention, measurement of frequency changes is carried out in an aqueous phase by a process which comprises hybridizing, followed by measuring frequency changes in water, removing the frequency conversion element with the immobilized DNA when frequency changes have reached a saturated state, washing with deionized water or an aqueous solution not containing the target DNA, and by measuring frequency changes.

In a preferable embodiment of the present invention, measurement of frequency changes is carried out under such temperature conditions that a hybridized DNA with the target DNA is not melted, but non-specific adsorption onto the frequency conversion element with the immobilized probe DNA is controlled.

In a preferable embodiment of the second embodiment of the present invention, the frequency conversion element with the immobilized DNA is dipped into water, followed by injecting the sample to detect the protein into the water to be adsorbed.

In a preferable embodiment of the second embodiment of the present invention, an aqueous sample to detect the protein is dropped in gaseous phase onto the frequency conversion element with the immobilized DNA to be adsorbed.

In a preferable embodiment of the second embodiment of the present invention, the DNA is immobilized in the form of a complex with a cationic lipid.

In a preferable embodiment of the second embodiment of the present invention, the DNA is selected from a group consisting of single-stranded DNA, double-stranded DNA and triple-stranded DNA.

In a preferable embodiment of the third and fourth embodiments of the present invention, the probe DAN is in the form of a complex with a cationic lipid, and the frequency conversion element is a crystal oscillator.

In the first embodiment of the present invention, measurement of frequency changes corresponding to mass changes of the frequency conversion element due to hybridization makes it possible to detect the formation of the hybridized DNA. The above detection of the formation of the hybridized DNA results in making it possible to detect a biochemical substance having a sequence complementary with the sequence of the probe DNA.

In a preferable embodiment of the first embodiment of the present invention, after frequency changes have reached a saturated state, the frequency conversion element is removed and washed with deionized water or with an aqueous solution not containing the target DNA, and frequency changes are then measured, resulting in that subjecting a mixture of a DNA having a sequence complementary with and specific to the probe DNA and a DNA not having the same to hybridization makes it possible to detect a hybridized DNA with only the DNA having a sequence complementary with the probe DNA, excluding the DNA not having a sequence complementary with the probe DNA and non-specifically and weakly adsorbed. In a preferable embodiment of the first embodiment of the present invention, measurement of frequency changes under such temperature conditions that the hybridized DNA with the target DNA is not melted, but non-specific adsorption onto the frequency conversion element with the immobilized probe DNA is controlled makes it possible to detect and identify only the hybridized DNA with the target DNA having a sequence complementary with the probe DNA. The above detection of the hybridized DNA results in making it possible to detect a biochemical substance having a sequence complementary with the probe DNA.

Firstly, the present invention makes it possible to provide a method of detecting biochemical substances, which is capable of simply and directly analyzing DNA hybridization reaction, and separating and determining hybridized DNA, and capable of accurately, quantitatively and simply hybridizing DNA to detect biochemical substances in an aqueous phase or gaseous phase, without the above disadvantages in the prior art, for example, requrements of special techniques, poor quantitative evaluation, taking a long time for measurements; and to provide a sensor used in the above method.

For example, since a sequence specific to RNA of a salmonella as a biochemical substance is solely capable of forming a hybridized RNA-DNA with a probe DNA having a sequence complementary with the sequence specific to the RNA of the salmonella, detection of the hybridized RNA-DNA from measurement of frequency changes corresponding to formation of hybridized RNA-DNA results in making it possible to detect the salmonella as the biochemical substance.

Secondly, the present invention makes it possible to provide a method of detecting biochemical substances, which is capable of simply and directly analyzing DNA hybridization reaction, determining binding constants on DNA hybridization to conduct kinetic analysis and separating and determining hybridized DNA, and capable of accurately, quantitatively and simply hybridizing DNA to detect biochemical substances, without the above disadvantages in the prior art, for example, requirements of special technique, poor quantitative evaluation, taking a long time for measurements, use of expensive apparatus, etc.; and to provide a sensor used in the above method.

Thirdly, the present invention makes it possible to provide a sensor for use in detection of biochemical substances, which sensor is applicable to both clinical examination and disease diagnosis by detection of DNAs specific to bacteria such as tubercle bacillus, virus such as AIDS virus, leukemia, canser, hereditary disease, etc.; food examination by the detection and determination of DNAs specific to Funji such as salmonellas contained in food; both assay and prification of intended DNA segments grown by cloning; synthesis of polynucleotides having an intended sequence; detection of proteins such as lac. repressor protein known as a DNA helical unstabilized protein, a T antigen produced from a monkey virus, and the like; detection of DNAs forming triple-helical structure; and the like.

Fourthly, the present invention makes it possible to provide a method of detecting biochemicals, which method is applicable to following of DNA hydrolysis reaction, evaluation of hydrolysis controlling effect, analysis of concentration effect of DNase and salt concentration effect of a measuring solution, improvement in efficiency of intercellular migration of a nucleic acid of gene, nucleic acid compounds such as ribozyme and antisense as interested from the standpoint of pharmacenticals, controlling of intercellular distribution thereof, and the like.

Fifthly, the present invention makes it possible to provide a method of detecting biochemicals, which method is applicable to analysis of characteristics specific to biochemical substances, for example, strength of hybridized DNA, effects of salt concentration of measuring solution, observation of intercalation phenomena etc., improvement in efficiency of intercellular migration of a nucleic acid of gene, nucleic acid compounds such as

ribozyme and antisense as interested from the standpoint of pharmaceuticals, controlling of intercellular distribution thereof, designing of medicinal substances, screening of medicinal substances, and the like.

The present invention are explained more in detail by the following Examples and Conparative Examples.

## Example 1

A DNA oligomer having 10 mer bases of 5'-dGGGAATTCGT-3' was synthesized by use of a DNA synthesizer according to phosphoramidite method to be used as a probe DNA. A thiol group was introduced at the 5'-phosphate end to be immobilized onto a gold electrode (16 $mm^2$) crystal oscillator, which is such that a frequency decrease of 1 Hz corresponds to a mass increase of about 1 ng both in aqueous solutions and in air. An aqueous solution of 5nM of the probe DNA with the thiol group was dropped onto the crystal oscillator in air, followed by reacting for a predetermined period of time, washing the aqueous solution on the gold electrode with deionized water, and by drying to immobilize the probe DNA onto the gold electrode. Immobilized amounts were determined from frequency changes. As shown in Table 1, 200 ng was immobilized in one hour, and 750 ng in 10 hours.

Separately, frequency changes due to hybridization with a target DNA were measured by use of the above crystal oscillator with the immobilized probe DNA of 150 to 200 ng in the same manner as above.

Onto the crystal oscillator with the immobilized probe DNA was dropped in air 6 pmol of a cyclic single-stranded M13DNA (MW=$2.4x10^6$ , 7249 base pairs) from Ecoli JM109 as a target DNA and having a sequence complementary with the probe DNA to be reacted, followed by washing the crystal oscillator with distilled water, drying in air, and by measuring frequency changes corresponding to mass changes due to hybridization. The amounts of frequency changes after drying and adsorbed amounts due to hybridization with the target DNA were shown in Table 2. Since measurement of frequency changes was carried out by use of such a crystal oscillator that adsorption of 1 ng of the target DNA results in a frequency change of 1 Hz, adsorbed amount of the target DNA due to hybridization was determined from frequency changes to be 600 ng as shown in Table 2.

## Comparative Example 1

The procedures of Example 1 were repeated except that a non-complementary target DNA obtained by removing a sequence complementary with the probe DNA from the target DNA in Example 1 was used in place of the target DNA in Example 1 with the result that no frequency changes were observed and it was impossible to detect the formation of hybridized DNA as shown in Table 2.

The comparison of the results of Example 1 with the results of Comparative Example 1 shows that it is made possible from frequency changes of the crystal oscillator to solely and specifically detect the M13DNA having the sequence complementary with the probe DNA.

Table 1

| | Reaction time | Frequency changes | Probe-immobilized amount |
|---|---|---|---|
| Example 1 | 1 hour | 200 Hz | 200 ng |
| | 10 hours | 750 Hz | 750 ng |

Table 2

| | Target DNA | frequency changes | Adsorbed amount |
|---|---|---|---|
| Example 1 | M13(Complementary M13DNA) | 600 Hz | 600 ng |
| Comparative Example 1 | M13 (non-complementary M13DNA) | 0 Hz | 0 ng |

Example 2

A single-stranded probe DNA with a thiol group as shown in Fig. 1 was prepared as follows.

A synthesized 10-mer probe DNA (MW=1480) having a sequence of 5'-dGGGAATTCGT-3' and having SH group at 5' phosphate end was obtained from Nippon Bioservice Co., Ltd. in a dry state, 0.5 ml of distilled water was added thereto to form an aqueous solution, followed by keeping under freezing little by little under nitrogen atmosphere. A synthesized 10-mer DNA (MW=1362) having a sequence of 5'-dACGAATTCCC-3' complementary with the probe DAN was obtained from Nippon Bioservice Co., Ltd. as a target DNA in a dry state, followed by preparing an aqueous solution, and by keeping under freezing little by little. After reverting to an aqueous solution at room temperature respectively, both solutions were mixed so that both probe DNA and target DNA may contain 3 $\mu$ g in 5.5 ml respectively. The resulting solution was kept at 4°C over one night so that a complete double helical structure may be formed. In order to prevent SH group from being oxidized by oxygen in water, the above solution was prepared by use of deacrated water, which was obtained by removing air bubbles by sucking with an aspirator applying an ultrasonic wave.

The same crystal oscillator as in Example 1 was dipped into the above aqueous solution at 17°C, and a time course of an immobilized amount of the probe DNA was measured with the result that the immobilized amount of the probe DNA reached a saturated state in an amount of about 900 ng in a dipping time of 3 hours as shown in Fig. 2. At a dipping time of 4 hours, the double-stranded probe DNA-immobilized crystal oscillator was dipped into a distilled water at 80°C higher than the melting temperature of the double-stranded probe DNA with the result that the immobilized amount onto the crystal oscillator was reduced to about 500 ng at a dipping time of 5 hours to show that a complementary DNA not having been chemically bound onto the gold electrode of the crystal oscillator of the double-stranded DNA was removed.

Example 3

A thiol group was introduced onto salmon testes DNA by the phosphoramidite method, and the same crystal oscillator as in Example 1 was dipped into an aqueous solution of the above DNA with thiol group to immobilize the DNA in an amount of 50 ng onto the gold electrode.

Complexes of the DNA immobilized onto the gold electrode with three synthesized cationic lipids, i.e. $2C_4N^+$, $2C_6N^+$ and $2C_8N^+$ having different carbon chain length respectively were prepared by mixing in an aqueous phase respectively. Three different DNA-complex-immobilized crystal oscillators were dipped into an aqueous solution at 25°C respectively, followed by injecting DNase 1 as a hydrolysis enzyme, measuring a time course of frequency changes corresponding to mass changes due to hydrolysis of the DNA immobilized onto the cyrstal oscillator by DNase 1, determining hydrolyzed amount from the time course of frequency changes, and determining DNA cleavage (%) as a percentage to a mass prior to measurement. The results are shown in Fig. 3.

The results show that the DNA not forming a complex with the cationic lipid was completely hydrolyzed in about 2 hours, whereas hydrolysis was controlled in the case of the DNA-complex, particularly no hydrolysis was observed in the case of the complex of DNA with $2C_8N^+$.

Example 4

The same probe DNA as in Example 1 was used. The same crystal oscillator as in Example 1 was used. The crystal oscillator was dipped into 10.6 nM probe DNA aqueous solution, followed by reacting for a predetermined period of time, removing the crystal oscillator from the aqueous solution, and by drying to immobilize the probe DNA onto the gold electrode. An immobilized amount of the probe DNA was determined from frequency changes. The results are shown in Fig. 4, in which 10 hours dipping results in a saturated state to immobilize 1500 ng. Fig. 4 is a graph showing a relationship between dipping time and dry weight of immobilized probe DNA at 25°C . Specific occupying area per the probe DNA molecule was determined to be 0.5 $nm^2$ at that time. The above value of the specific occupying area is considered to be reasonable compared with a theoretical value in the case of adsorption as a monomolecular film, i.e., 0.15 $nm^2$. As explained later, since the target DNA to be adsorbed onto the probe DNA has such a large molecular weight as to be $2.4 \times 10^6$, in actual experiments, the crystal oscillator was dipped for about 20 to 30 minutes to immobilize 150 to 200 ng of the probe DNA and to be used.

Next, hybridization of the target DNA onto the cyrstal oscillator with immobilized probe DNA was measured. The same target DNA as in Example 1 was used. For comparison, a non-complementary DNA obtained by removing EcoRi binding sequence from the M13DNA was used as a control to compare adsorption behavior therebetween. As shown in Fig. 5, M13DNA having the complementary sequence was added at a time shown

by arrow marks in an amount of 0.125 p mol respectivly, resulting in that as the complementary DNA is added, frequency is reduced to show that the M13DNA is bound with time. As the added amount is increased, frequency reduction reached a saturated state to be a frequency decrease of about 600 Hz, resulting in that about 600 ng of the M13DNA was bound, because such a crystal oscillator that a frequency change of 1 Hz corresponds to a mass change of about 1 ng is used, and in that the above frequency changes is consistent with frequency changes when dried in air. The above results show that under the above conditions, about half of the M13DNA having the complementary sequence and injected into the aqueous solution has been bound with the probe DNA. The crystal oscillator was removed from the aqueous solution and was washed with deionized water or with an aqueous solution not containing the target DNA with the result that the target DNA was hardly removed. On the other hand, the M13DNA not having the complementary sequence was injected into the aqueous solution in the same manner as above with the result that adsorption with time is observed as shown in Fig. 6, but when the resulting crystal oscillater is dipped into or washed with deionized water or an aqueous solution not containing the target DNA, the adsorbed non-complementary DNA was completely removed. The above result shows that the M13DNA not having the complementary sequence may non-specifically and weakly be adsorbed onto the probe DNA at 25°C . In order to avoid such non-specific adsorption, dependence of hybridization on temperature was measured. Fig. 5 is a graph showing a time course of binding of the' complementary M13DNA to the probe DNA at 25 °C . Fig. 6 is a graph showing a time course of binding of the non-complementary DNA to the probe DNA at 25°C . In Fig. 5, a bound amount of the complementary M13 DNA was 550 ng as dry weight.

The crystal oscillator with immobilized probe DNA was dipped into an aqueous solution containing M13DNA, and the temperature of the aqueous solution was slowly raised to measure frequency changes with varied temperatures. The results are shown in Fig. 7 and Fig. 8 respectively. Fig. 7 shows actually measured frequency changes, while Fig. 8 shows frequeny changes calibrated by deducting changes due to temperature dependence of the standard frequency of the crystal oscillator from frequency changes with varied temperatures on hybridization. The results in Fig. 7 and Fig. 8 show that in the case of the complementary M13DNA, a melting temperature (Tm) is observed at 60°C , the complementary M13DNA is hybridized and strongly bound with the probe DNA at a temperature below about 60°C but as the temperature is increased above 6O°C , the hybridized DNA melts, resulting in increasing in frequency and decreasing in weight. On the other hand, in the case of the non-complementary M13DNA, as the temperature increases, frequency slowly increases, resulting in not showing a clear melting temperature (Tm). The above results show that both the complementary M13DNA and non-complementary M13DNA are adsorbed in experiments at 25°C , and that it is necessary to carry out adsorption experiments around 50°C in order to distringuish the complementary M13DNA from the non-complementary M13DNA on adsorption. In Fig. 7, a mark Δ shows the case of the crystal oscillator alone or the crystal oscillator with immobilized probe DNA alone.

Separately, regarding to the melting temperature (Tm) of the M13DNA having a sequence complementary with the probe DNA, absorbance changes of ultraviolet spectrum during increasing temperature and decreasing temperature were measured with the result that the melting temperature (Tm) was confirmed to be 60°C as shown in Fig. 9. Fig. 9 is a graph showing a relationship between temperature and absorbance in a melting curve obtained by ultraviolet absorption on the M13DNA hybridized with the probe DNA.

From the results of the melting curve on hybridization of the M13DNA having a sequence complementary with the probe DNA, adsorption behaviors of the M13DNA having the sequence complementary with the 10-mer probe DNA and of the M13DNA not having the complementary sequence were examined in an aqueous solution at 50°C . Fig. 10 shows a graph showing the results of adsorption experiments at 25°C. Fig. 11 shows a graph showing the results of adsorpiton experiment at 50°C . The results at 50°C shows that non-specific adsorption of the M13DNA not having the sequence complementary with the probe DNA is controlled and only the M13DNA hybridized with the probe DNA is solely bound. As above described, use of the crystal oscillator as a device makes it possible to determine hybridized DNA in the order of ng, and to observe a time course of binding on hybridization. In Fig. 10 and Fig. 11, the complementary M13DNA and the non-complementary M13DNA were added in an amount of 0.124 p mol (360 ng) at a time shown by an arrow mark respectively.

## Example 5

Hybridization experiments were carried out in the same manner as in Example 4 except that a target DNA molecule (5'-dACGAATTCCC-3') having substantially the same molecular weight as the probe DNA in Example 4 and consisting of 10 bases complementary from the 3' end and a comparative target DNA molecule (5'-dGGGAATTCGT-3') partly complementary from the 3' end were used in place of the target DNA in Example 4. The results are shown in Fig. 12, in which as the complementary target DNA molecule is added and increased in concentration, frequency is decreased to show the formation of a hybridized DNA molecule, whereas no frequency changes were recognized in the case of the non-complementary DNA molecule (5'-dGGGAATTCGT-3'). The above results show that frequency changes due to specific hybridization are clearly distinguished from frequency changes due to the target DNA molecule non-complementary with the probe DNA.

In Fig. 12, an immobilized amount of the probe DNA was 847 ng. At a time shown by an arrow mark, 1.5 p mol (45 ng) of the complementary 10-mer target DNA molecule or of the non-complementary 10-mer target DNA molecule was added respectively. The bound amount of the complementary 10-mer target DNA molecule was finally 300 ng on the dry basis. Fig. 12 is a graph showing a time course of frequency changes due to binding of the complementary or non-complementary 10-mer target DNA molecule onto the probe DNA.

Example 6

For the purpose of studying specificity of sequence, binding strength thereof, etc., targets comprising nucleotides having miss-matched bases as shown in Table 3 were synthesized, and frequency changes due to hybridization were analyzed at 25°C in the same manner as in Example 4 by use of the crystal oscillation used in Example 4. The results showed that nucleotides having continuous eight or more complementary base pairs are bound and recognized, whereas such nucleotide as to have a miss-matched base at the center is' not bound not to be recognized. Dependency of frequency changes for such a nucleotide as to have a miss-matched base at the center on temperature was studied with the result that a clear melting temperature (Tm) was not recognized as shown in Fig. 13, and that specific adsorption has not been occurred. The above results are consistent with the results of analysis of hybridization according to the conventional method of recognizing decrease of the melting temperature (Tm) by measurement of ultraviolet spectrum absorbance. From the above results, the method by use of the crystal oscillator makes it possible to more simply follow a time course of hybridization compared with the conventional method, and is advantageous as a means to analyze temperature characteristics of hybridization, binding strength thereof, kinetics thereof, etc.

Fig. 13 is a graph showing a relationship between frequency changes and temperature regarding to temperature effects of 3'-d-CCCTTAAGCA-5' nucleotide in Table 3 or 3'-d-CCCTAAAAGCA-5' nucleotide having a miss-matched base at a concentration of 1 μ g or 3.3 n mol in 5 ml and temperature respectively.

Table 3

| Nucleotides | Bound amount (ng) | Hybridization percentage (%) |
|---|---|---|
| 3' dCCCTTAAGCA5' | 380± 10 | 100 |
| 3' dCCCTTAAGGG5' | 350± 10 | 92 |
| 3' dTGCTTAAGCA5' | 350± 10 | 92 |
| 3' dCCCTAAAGCA5' | 125± 10 | 31 |
| 3' dCCCTGAAGCA5' | 100± 10 | 26 |
| 3' dCTGCTACGGG5' | 0 | 0 |
| 3' dAGCCGTACCC5' | 0 | 0 |

Concentration: 400 ng or 130 p mol in 10 ml.
Underlines show complementary base pairs.

Example 7

Adsorption experiments were carried out in the same manner as in Example 4 regading to the effect of immobilized amounts on binding amounts of target M13DNA in Example 4 in the case where the probe DNA in Example 4 was immobilized in the same manner as in Example 4. The results are shown in Fig. 14 and Fig. 15.

Fig. 14 is a graph showing a time course of frequency changes regarding to the effect of the immobilized amount of the probe DNA on the binding amount of the complementary M13DNA. In Fig. 14, a curve (1) shows a case where the immobilized amount is 1400 ng or 470 p mol, and a curve (2) shows a case where the immobilized amount is 106 ng or 35 p mol. The amount of the complementary M13DNA injected into the aqueous solution, into which the probe-immobilized crystal oscillator is dipped, was 480 ng or 20 p mol respectively.

Fig. 15 is a graph showing a relationship of the immobilized amount of the probe DNA with the binding amount of the complementary M13DNA or with percentage of the complementary M13DNA bound against the complementary M13DNA added.

The results in Fig. 14 show that the amount of the complementary M13DNA to be hybridized varies depending on the immobilized amount of the probe DNA. The results in Fig. 15 show that as the immobilized amount of the probe DNA is decreased, the amount of the complementary M13DNA to be hybridized is increased. It is considered that a smaller amount of the probe DNA to be immobilized makes it possible to more efficiently hybridize the complementary M13DNA.

Example 8

Experiments were carried out on a relationship of a specific binding amount onto the probe DNA in Example 4 with the complementary M13DNA in Example 4 and with the target DNA molecule (10-mer) in Example 5. The results are shown in Fig. 16 and Fig. 17.

Fig. 16 is a graph showing a relationship between the amount of the complementary M13DNA in 10 ml of the aqueous solution and the binding amount of the complementary M13DNA, wherein the amount of the probe DNA was 108 ng. In Fig. 16, a molecular ratio of the hybridized complementary M13DNA to the probe DNA at a point shown by an arrow mark and with a saturated binding amount was 1:200.

Fig. 17 is a graph showing a relationship between the 10-mer DNA amount in 10 ml and the binding amount of the 10-mer DNA, wherein the amount of the probe was 847 ng. In Fig. 17, a molecular ratio of the hybridized 10-mer DNA to the probe DNA at a point shown by an arrow mark and with a saturated binding amount was 1:2.

The result in Fig. 16 show that one molecule of the complementary M13DNA is adsorbed per 200 molecules of the probe DNA. The results in Fig. 17 show that one molecule of the target DNA is adsorbed per 2 molecules of the probe DNA. The above results show that hybridization takes place quantitatively with reproducibility.

Examples 9-11

The same crystal oscillator used in Example 1 was dipped into an aqueous solution in which a DNA oligomer having 10 bases (5'-dGGGAATTCGT-3') with thiol group at the 5' phosphate end and a DNA oligoner not having thiol group and having 10 bases (3'-CCCTTAAGCA-5') complementary with the first DNA oligoner have been hybridized, to be immobilized onto the gold electrode. A complementary strand was removed by aging, followed by removing the crystal oscillator from the aqueous solution, dipping the crystal oscillator with immobilized DNA oligomer of 500 ng into 5 ml of deionized water, injecting 500 ng of a DNA oligomer complementary with the DNA oligomer immobilized onto the crystal oscillator to be hybridized until an adsorption equilibrium is reached, removing the crystal oscillator, dipping the crystal oscillator into a large amount of deionized water, and by measuring a time course of frequency changes at 20°C (Example 9), 30°C (Example 10) and 6O°C (Example 11) respectively.

Fig. 18 shows the results at 20°C (Example 9).

For the purpose of studying an effect of temperature on hybridization of DNA, binding constants of DNA double-helical structure at respective measuring temperatures were calculated. For example, a binding constant at 20°C was determined in reference to Fig. 18 as follows. In Fig. 18, an initial adsorption rate $V_1$ corresponding to hybridization was determined from a slope of a tangential line of a hybridization curve at the beginning, followed by determining a binding rate constant $k_1$ from the above $V_1$ by the application of the equations described above, determining an initial dissociation rate $V_2$ from a slope of a tangential line at the beginning of a dissociation curve obtained by dipping the crystal oscillator into a large amount of deioorized water, determining a dissociation rate constant $k_2$ from the above $V_2$ by the application of the equations described above, and determining a binding constant (K) at 20°C from the binding rate constant $k_1$ and the dissociation rate constant $k_2$ by the application of the equations described above. The experiments at 20°C (Example 9) were repeated except that the measuring temperature was varied to be 30°C (Example 10) and 60°C (Example 11) respectively. The results are shown in Table 4.

Table 4

| | Temperature/°C | $k_1/M^{-1}s^{-1}$ | $k_{-2}/10^{-5}s^{-1}$ | $K/10^5M^{-1}$ |
|---|---|---|---|---|
| Example 9 | 20 | 1600 | <2.8 | 570 |
| Example 10 | 30 | 490 | 14 | 35 |
| Example 11 | 60 | 790 | 240 | 3.4 |

Example 12

Into 5.5 ml of deionized water kept at 60°C was dipped the same crystal oscillator with immobilized probe DNA as in Example 9, after reaching a steady state for measuring frequency, 1800 ng of the same target DNA as in Example 9 was injected, and a saturated adsorbed amount ($\Delta$ m) of the target DNA when equilibrium reached was determined. The same procedures as above were repeated respectively except for varying the injected amount to be 1800 ng, 3600 ng and 5400 ng respectively to obtain a relationship between a concentration of the target DNA and a saturated adsorbed amount ($\Delta$ m) of the target DNA as shown in Fig. 19. Since the saturated adsorbed amount is much smaller compared with the injected amount of the target DNA, the above relationship substantially corresponds to a relationship between an initial concentration $[target]_0$ of the target DNA and $\Delta$ m. Thus, the relationship obtained from Fig. 19 was applied to the aforementioned formula (I) to take a concentration of the target DNA on X axis and concentration of target DNA/$\Delta$ m on Y axis as substantially corresponding to a relationship between $[target]_0$ and $[target]_0/\Delta$ m, and by plotting. The results are shown in Fig. 20. A'saturated adsorbed amount ($\Delta m_{max}$) of the target DNA onto the probe DNA was determined from a slope of a straight line through the above plots, and a binding constant (K) obtained from a value of a Y axis intercept of the above straight line in Fig. 19 was 3.4 x $10^9$ $M^{-1}$.

## Claims

1. A method of detecting biochemical substances which method comprises chemically binding and immobilizing a probe DNA having a sequence complementary with a sequence specific to a DNA of a biochemical substance onto an electrode of a frequency conversion element, hybridizing the probe DNA with a sample to detect said DNA as a target DNA having a sequence specific to the probe DNA, and measuring frequency changes corresponding to mass changes due to hybridization to detect and identify a hybridized DNA.

2. A method of detecting biochemical substances which method comprises chemically binding and immobilizing a DNA constituting a biochemical substance and specifically adsorbing a protein as a biochemical substance onto an electrode of a frequency conversion element, adsorbing a sample to detect the protein, and measuring frequency changes corresponding to mass changes due to adsorption onto the electrode to detect the protein.

3. A method as claimed in claim 1 or 2, wherein said frequency conversion element is a crystal oscillator.

4. A method as claimed in claim 1, wherein an aqueous sample to detect said DNA is dropped onto the frequency conversion element with the immobilized probe DNA in a gaseous phase to be hybridized.

5. A method as claimed in claim 1, wherein the frequency conversion element with the immobilized probe DNA is dipped into water, followed by injecting the sample to detect the DNA into the water to be hybridized.

6. A method as claimed in claim 1, wherein the probe DNA is a single-stranded DNA and the frequency conversion element with the immobilized DNA is dipped into water, followed by injecting into the water a sample DNA molecule selected from a group consisting of a DNA molecule having substantially the same number of bases or less, substantially the same molecular weight or less and at least one base pair specific to the probe DNA, a DNA molecule having substantially the same number of bases or less, substantially the same molecular weight or less and having no base pairs specific to the probe DNA and mixtures thereof, as a target DNA molecule to be hybridized with the probe DNA, and measuring frequency changes corresponding to mass changes due to hybridization to detect and identify a hybridized DNA molecule.

**7.** A method as claimed in claim 1, wherein the probe DNA is a single-stranded DNA and the frequency conversion element with the immobilized probe DNA is dipped into water, followed by injecting into the water a sample DNA molecule comprising substantially the same number of bases as the probe DNA or less or comprising a nucleic acid component containing the above bases as a target DNA to be hybridized with the probe DNA, and measuring frequency changes corresponding to mass changes due to hybridization to detect and identify a hybridized DNA molecule.

**8.** A method as claimed in claim 1, wherein the frequency conversion element acid the immobilized probe DNA is dipped into water, followed by injecting a sample to detect the DNA into the water, hybridizing the probe DNA with the sample as a target DNA having a sequence specific to the probe DNA, and measuring frequency changes with time to determine a binding constant (K) on hybridization.

**9.** A method as claimed in claim 2, wherein the frequency conversion element with the immobilized DNA is dipped into water, followed by injecting the sample to detect the protein into the water to be adsorbed.

**10.** A method as claimed in claim 2, wherein an aqueous sample to detect the protein is dropped in gaseous phase onto the frequency conversion element with the immobilized DNA to be adsorbed.

**11.** A method as claimed in claim 1, wherein the electrode is a gold electrode and the probe DNA is immobilized onto the gold electrode by a process which comprises introducing a thiol group onto an end of the complementary sequence of the probe DNA, dipping the frequency conversion element into an aqueous solution of the probe DNA with the thiol group to be reacted and chemically bound, followed by removing the frequency conversion element and drying.

**12.** A method as claimed in claim 1, wherein the electrode is a gold electrode and the probe DNA is immobilized onto the gold electrode by a process which comprises introducing a thiol group onto an end of the complementary sequence of the probe DNA, dropping an aqueous solution of the probe DNA with the thiol group onto the frequency conversion element in a gaseous phase to be reacted and chemically bound, followed by washing with a deionized water or with an aqueous solution not containing any DNA to be hybridized with the probe DNA, and by drying.

**13.** A method as claimed in claim 1, wherein the electrode is a gold electrode and the probe DNA is single-stranded and immobilized onto the gold electrode by a process which comprises introducing a thiol group onto an end of the sequence of the probe DNA, hybridizing the probe DNA having the thiol group with a DNA having a complementary sequence to form a double-stranded DNA, dipping the frequency conversion element into an aqueous solution of the double-stranded DNA with thiol group to be chemically bound, heating at a melting temperature or higher of the double-stranded DNA to desorb a single-stranded DNA not having been chemically bound on the electrode, followed by removing the frequency conversion element, and drying.

**14.** A method as claimed in claim 1, wherein the electrode is a gold electrode and the probe DNA is single-stranded and immobilized onto the gold electrode by a process which comprises introducing a thiol group onto an end of the sequence of the probe DNA, hybridizing the probe DNA having the thiol group with a DNA having a complementary sequence to form a double-stranded DNA, dropping an aqueous solution of the double-stranded DNA with thiol group onto the frequency conversion element in a gaseous phase to be reacted and chemically bound, heating at a melting temperature or higher of the double-stranded DNA to desorb a single-stranded DNA not having been chemically bound on the electrode, followed by washing with a deionized water or with an aqueous solution not containing any DNA to be hybridized with the probe DNA, and by drying.

**15.** A method as claimed in claim 6, the DNA molecule having a sequence specific to the probe DNA is obtained by a process which comprises dipping the frequency conversion element with the immobilized probe DNA into an aqueous solution containing respective monomer nucleotides constituting the DNA molecule, arranging respective monomer nucleotides so as to form complementary base pairs with the probe DNA, and polymerizing the arranged monomer nucleotides in the presence of an enzyme.

**16.** A method as claimed in claim 1, wherein the DNA is double-stranded, the probe DNA is a single-stranded DNA constituting the double-stranded DNA, and the target DNA is another single-stranded DNA constituting the double-stranded DNA and complementary with the probe DNA.

**17.** A method as claimed in claim 1, wherein the DNA is triple-stranded, the probe DNA is a double-stranded DNA constituting the triple-stranded DNA, and the target DNA is another single-stranded DNA constituting the triple-stranded DNA and complementary with the probe DNA.

**18.** A method as claimed in claim 1, wherein the DNA is triple-stranded, the probe DNA is a single-stranded DNA constituting the triple-stranded DNA, and the target DNA is other double-stranded DNA constituting the triple-stranded DNA and complementary with the probe DNA.

**19.** A method as claimed in claim 1, wherein measurement of frequency changes is carried out in gaseous phase by a process which comprises hybridizing, followed by washing with deionized water or with an aqueous solution not containing the target DNA, and by drying.

**20.** A method as claimed in claim 1, wherein measurement of frequency changes is carried out in water by a process which comprises hybridizing, followed by measuring frequency changes in water, removing the frequency conversion element with the immobilized DNA when frequency changes have reached a saturated state, washing with deionized water or an aqueous solution not containing the target DNA, and by measuring frequency changes.

**21.** A method as claimed in claim 1, wherein measurement of frequency changes is carried out under such temperature conditions that a hybridized DAN with the target DNA is not melted, but non-specific adsorption onto the frequency conversion element with the immobilized probe DNA is controlled.

**22.** A method as claimed in claim 2, wherein the DNA is selected from a group consisting of single-stranded DNA, double-stranded DNA and triple-stranded DNA.

**23.** A method as claimed in claim 1, wherein the probe DNA is immobilized in the form of a complex with a cationic lipid.

**24.** A method as claimed in claim 2, wherein the DNA is immobilized in the form of a complex with a cationic lipid.

**25.** A sensor used in a method as claimed in claim 1, which sensor comprises a frequency conversion element and a probe DNA chemically bound and immobilized onto an electrode of the frequency conversion element and having a sequence complementary with a sequence specific to a DNA of a biochemical substance.

**26.** A sensor used in a method as claimed in claim 2, which sensor comprises a frequenoy conversion element and a DNA constituting a biochemical substance, specifically adsorbing a protein as a biochemical substance and being chemically bound and immobilized onto an electrode of the frequency conversion element.

**27.** A sensor as claimed in claim 25, wherein the probe DNA is in the form of a complex with a cationic lipid.

**28.** A sensor as claimed in claim 26, wherein the DNA is in the form of a complex with a cationic lipid.

**29.** A sensor as claimed in claim 25, 26,. 27 or 28, wherein the frequency conversion element is a crystal oscillator.

F I G. 1

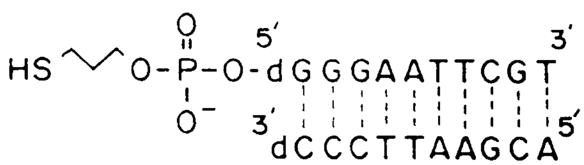

F I G. 2

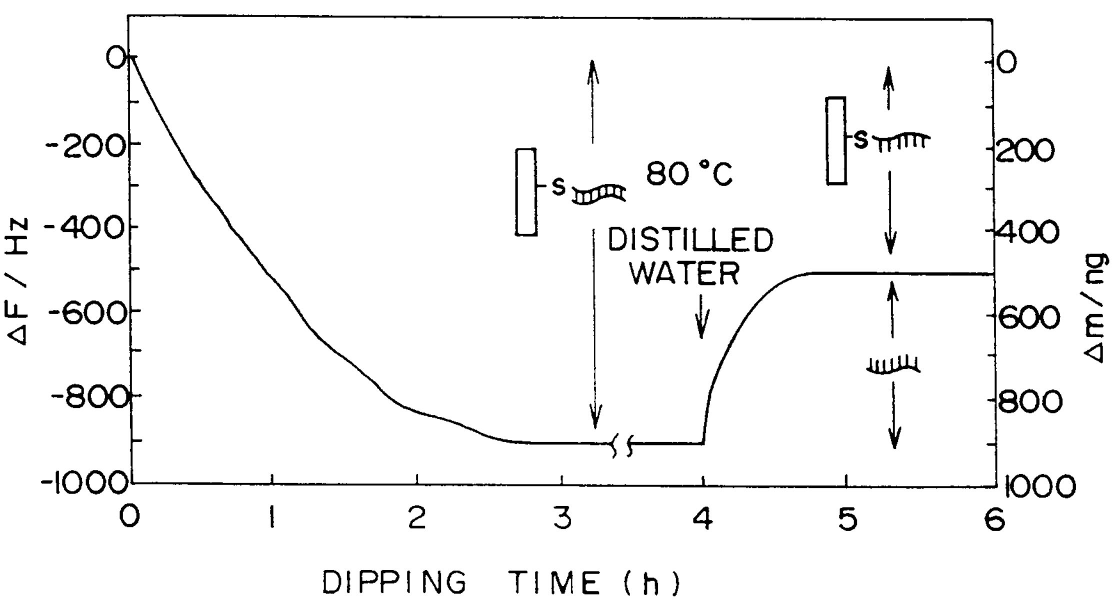
ΔF / Hz
0
-200
-400
-600
-800
-1000
Δm / ng
0
200
400
600
800
1000
80 °C
DISTILLED
WATER
0
1
2
3
4
5
6
DIPPING TIME (h)

FIG. 3

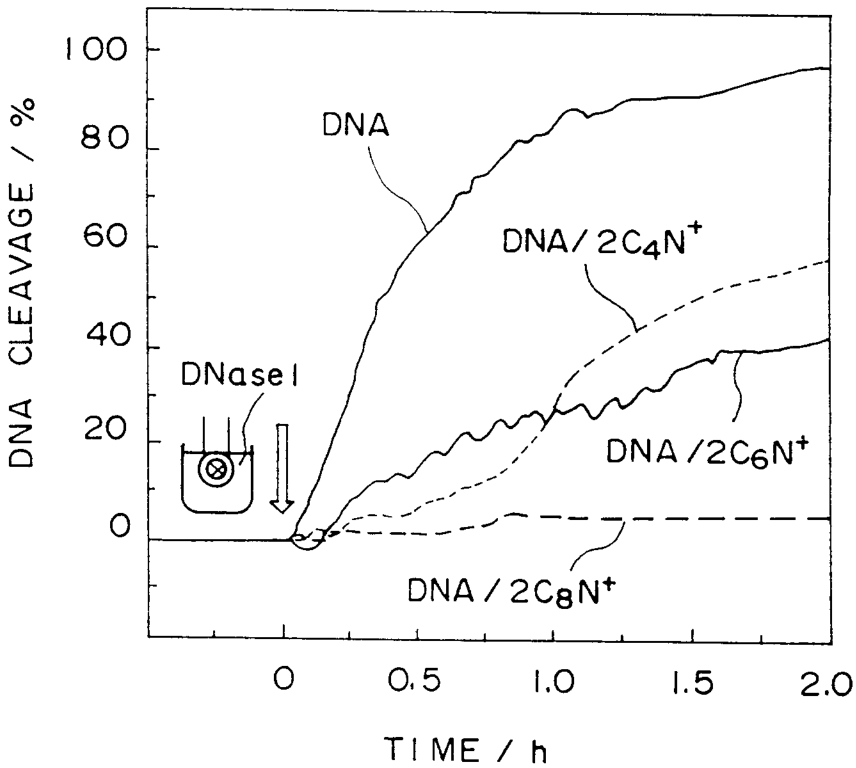
100
80
60
40
20
0
DNA CLEAVAGE / %
DNA
DNA/2C4N+
DNA/2C6N+
DNA/2C8N+
DNase I
0
0.5
1.0
1.5
2.0
TIME / h

FIG. 4

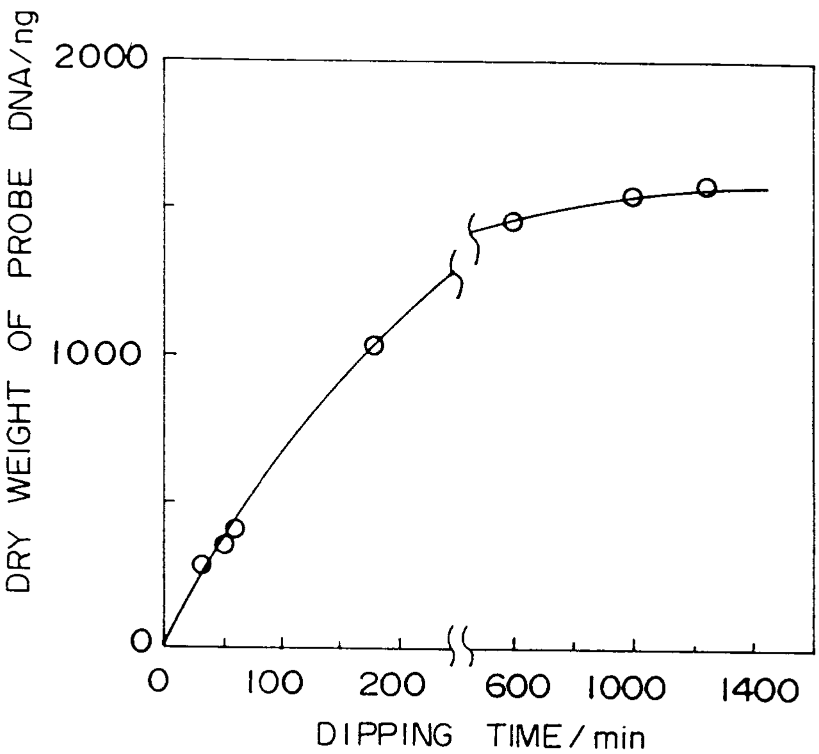
DRY WEIGHT OF PROBE DNA/ng
2000
1000
0
0
100
200
600
1000
1400
DIPPING TIME/min

FIG. 5

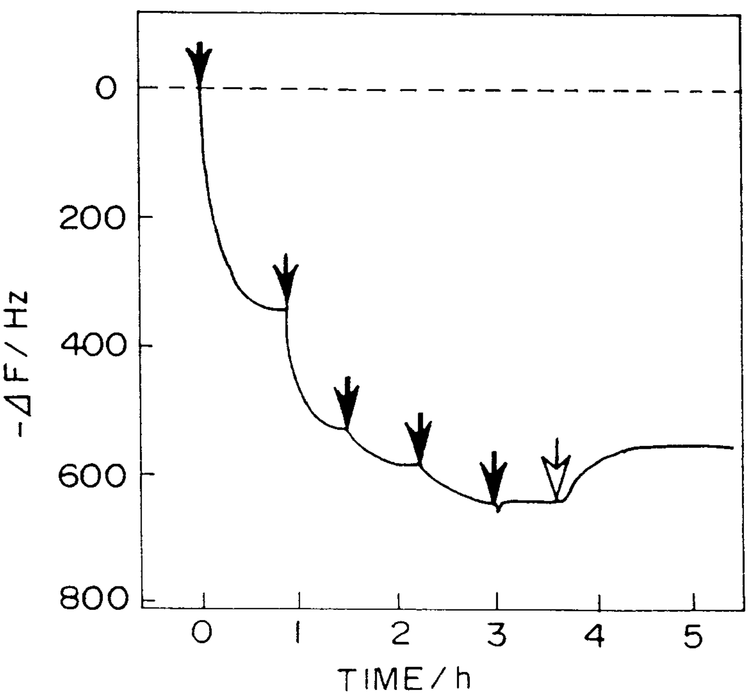
-ΔF/Hz
0
200
400
600
800
0
1
2
3
4
5
TIME/h

FIG. 6

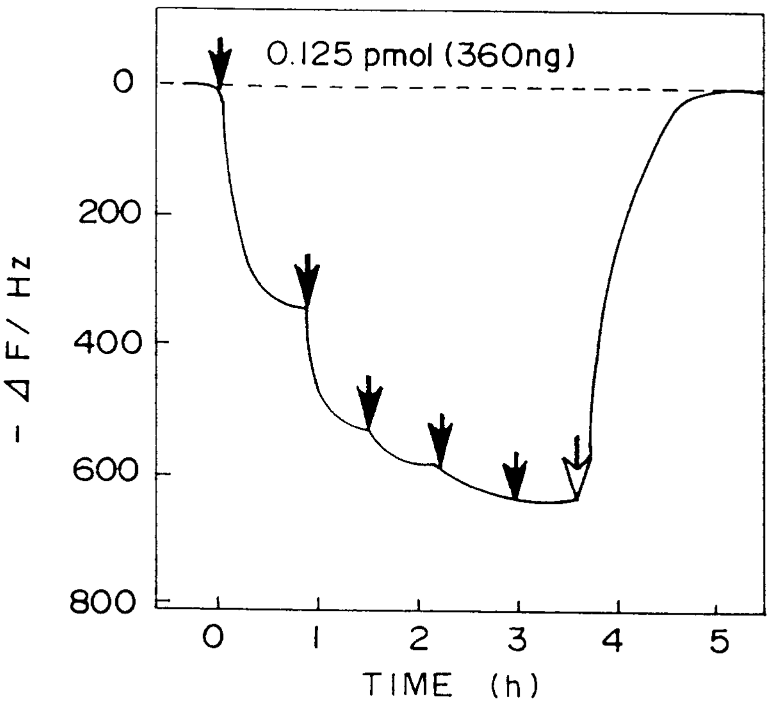
0.125 pmol (360ng)
- ΔF / Hz
0
200
400
600
800
0
1
2
3
4
5
TIME (h)

FIG. 7

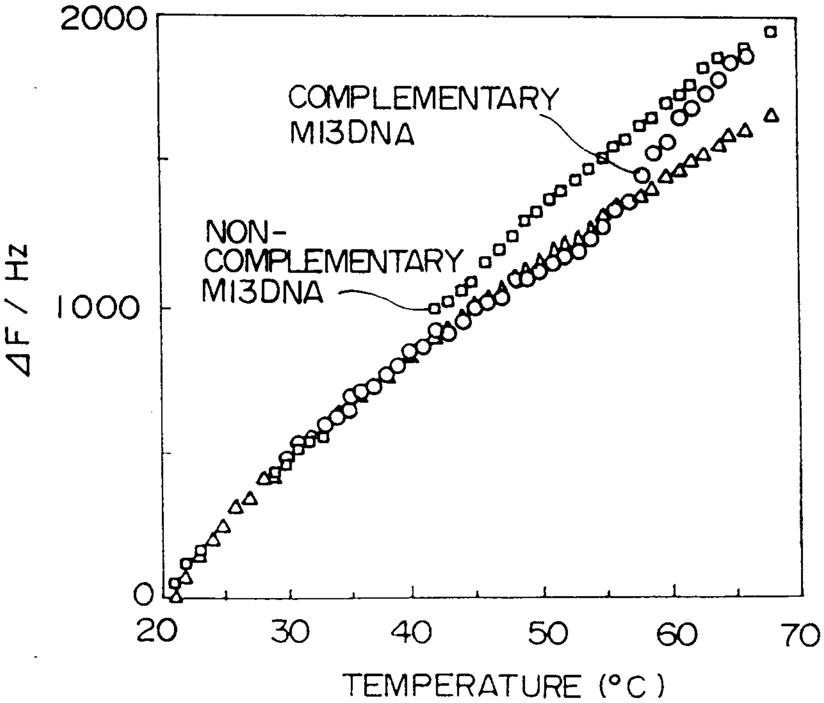
COMPLEMENTARY
M13DNA
NON-
COMPLEMENTARY
M13DNA
ΔF / Hz
2000
1000
0
20
30
40
50
60
70
TEMPERATURE (°C)

FIG. 8

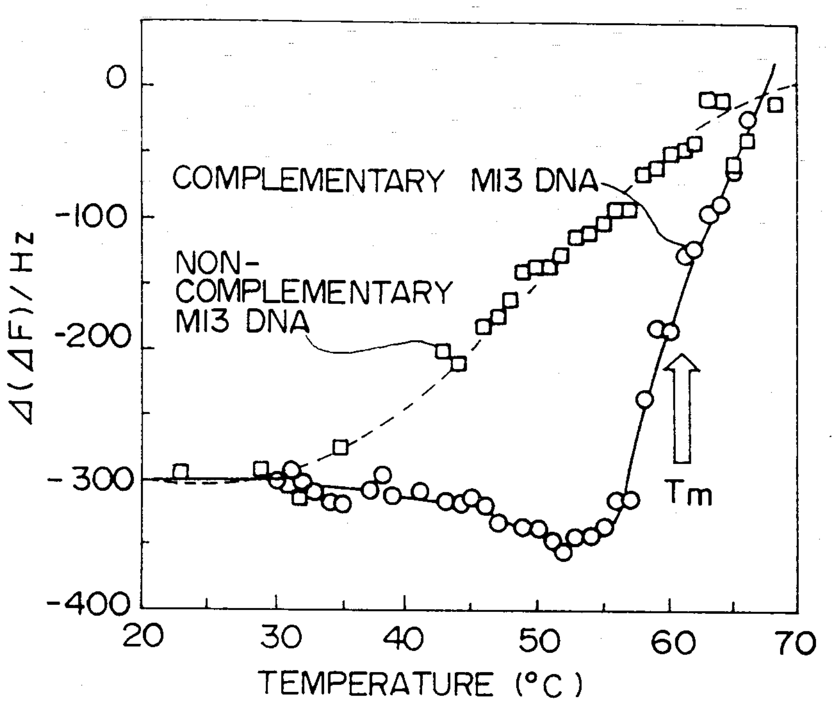
0
-100
-200
-300
-400
Δ(ΔF)/Hz
20
30
40
50
60
70
TEMPERATURE (°C)
COMPLEMENTARY M13 DNA
NON-COMPLEMENTARY M13 DNA
Tm

FIG. 9

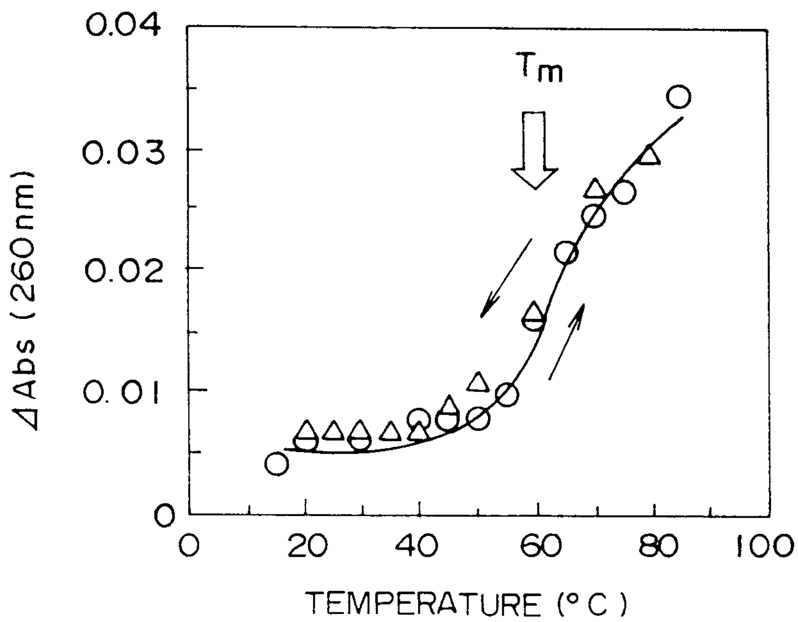
0.04
0.03
0.02
0.01
0
ΔAbs (260nm)
0
20
40
60
80
100
TEMPERATURE (°C)
Tm

# FIG. 10

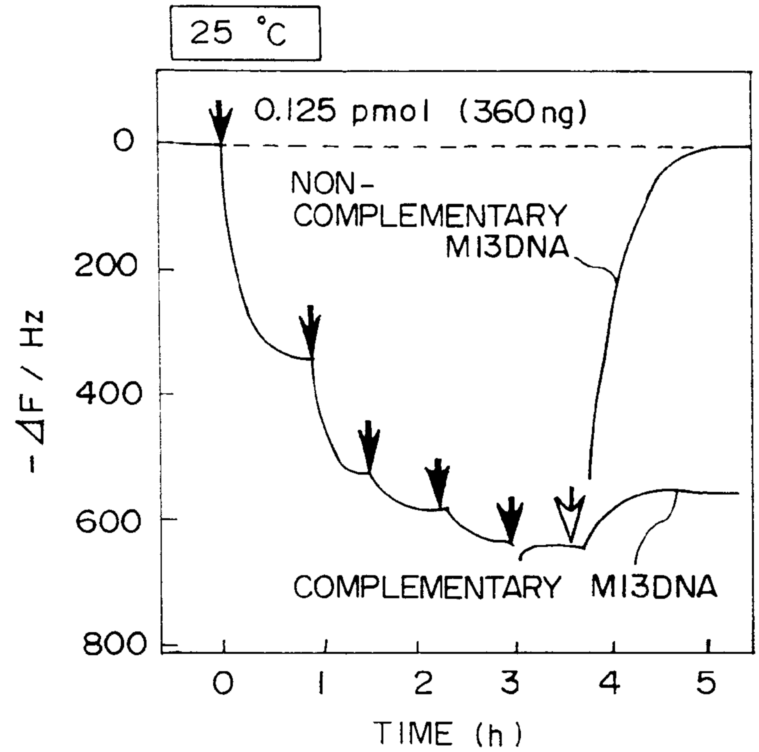
25 °C
0.125 pmol (360 ng)
NON-
COMPLEMENTARY
M13DNA
COMPLEMENTARY M13DNA
-ΔF / Hz
0
200
400
600
800
0
1
2
3
4
5
TIME (h)

# FIG. 11

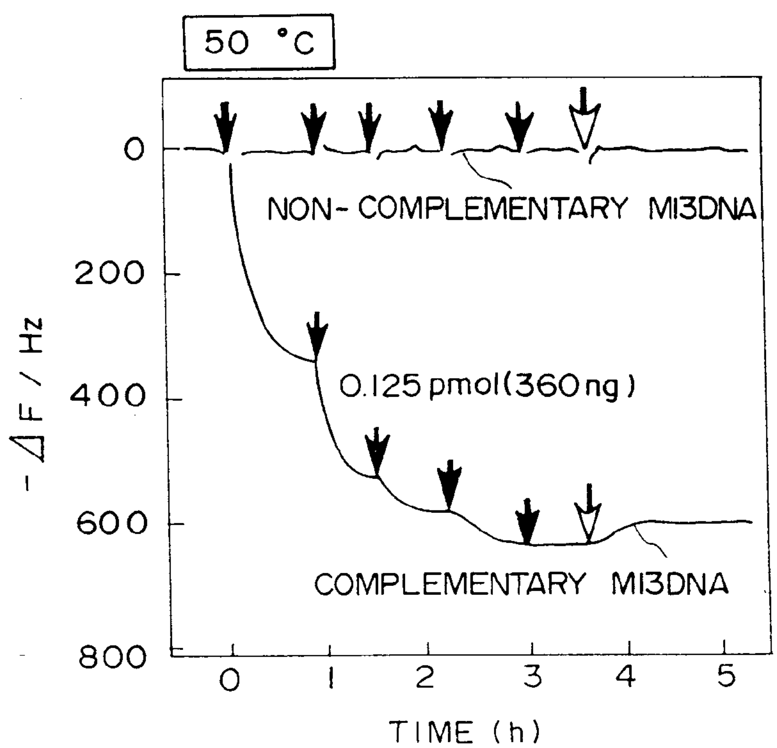
50 °C
NON-COMPLEMENTARY M13DNA
0.125 pmol(360 ng)
COMPLEMENTARY M13DNA
-ΔF / Hz
0
200
400
600
800
0
1
2
3
4
5
TIME (h)

FIG. 12

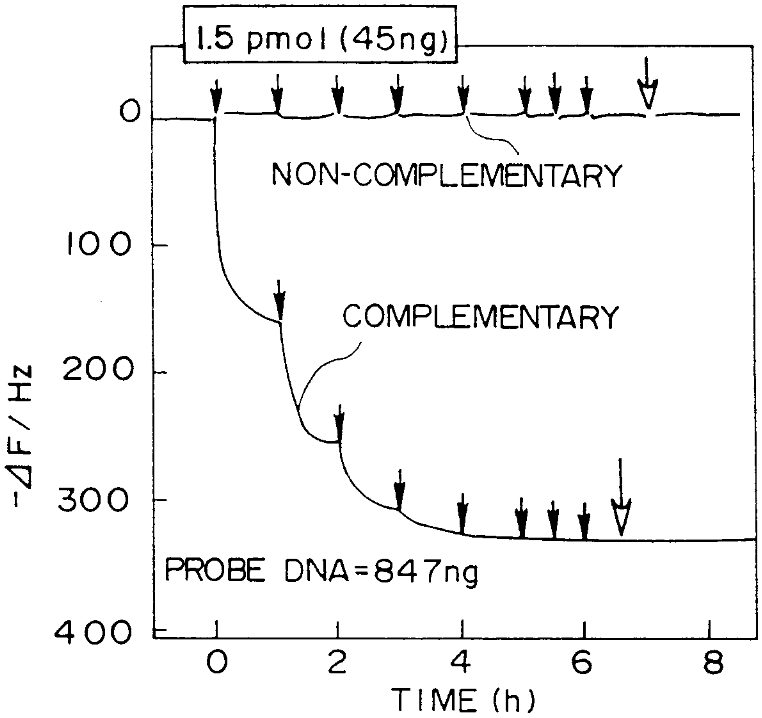

1.5 pmol (45ng)
0
100
200
300
400
-ΔF / Hz
NON-COMPLEMENTARY
COMPLEMENTARY
PROBE DNA=847ng
0
2
4
6
8
TIME (h)


FIG. 13

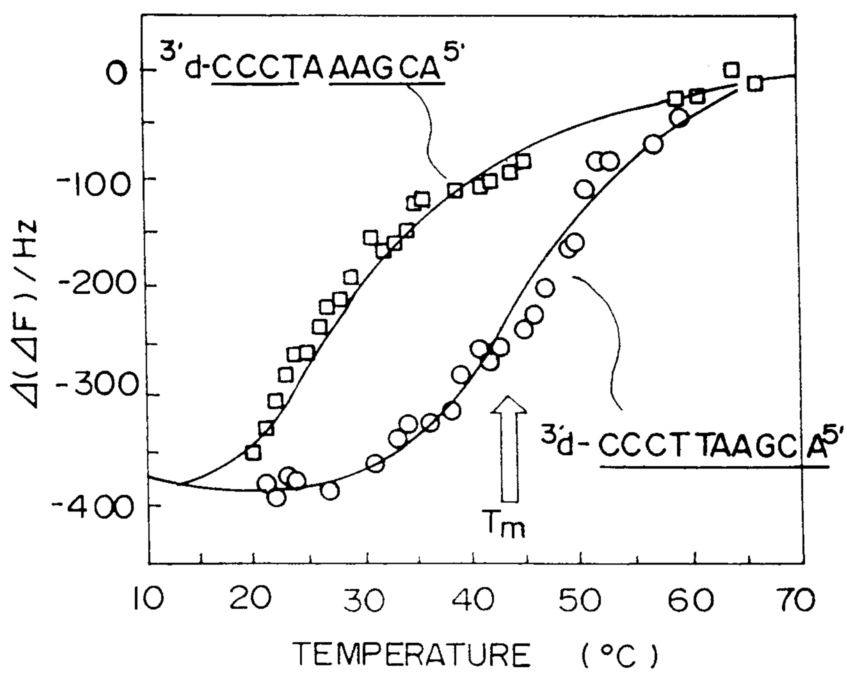

3'd-CCCTAAAGCA5'
3'd-CCCTTAAGCA5'
Tm
0
-100
-200
-300
-400
Δ(ΔF) / Hz
10
20
30
40
50
60
70
TEMPERATURE (°C)

F I G. 14

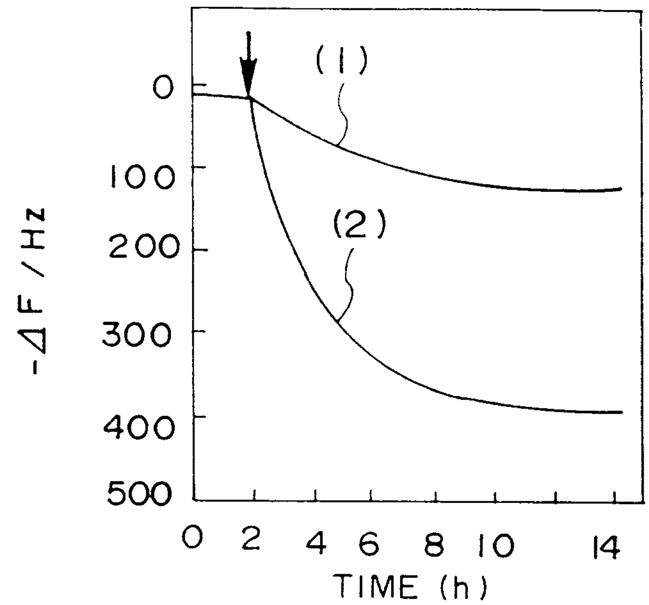
(1)
(2)
-ΔF / Hz
0
100
200
300
400
500
0
2
4
6
8
10
14
TIME (h)

F I G. 15

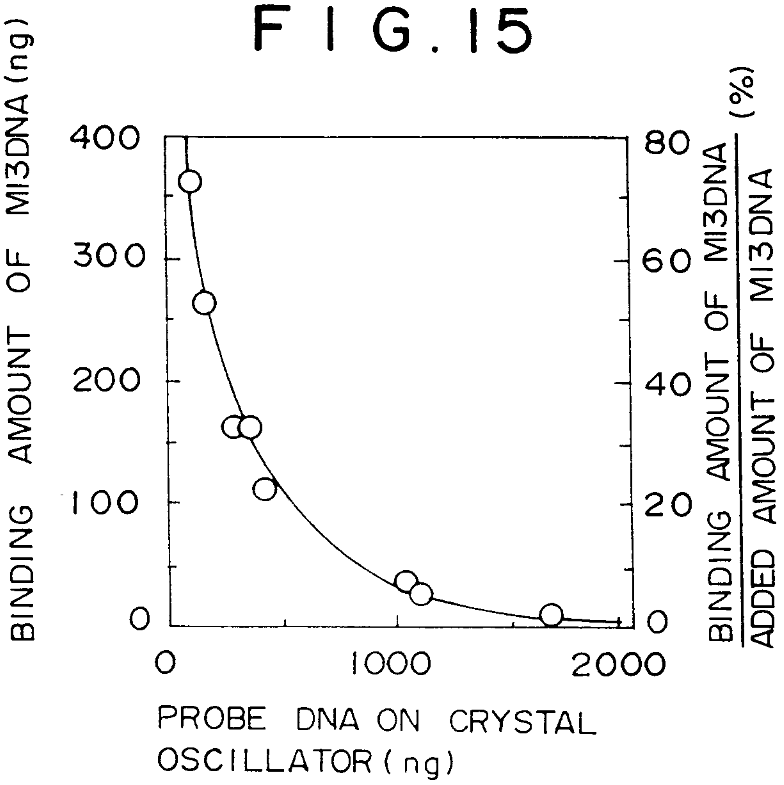
BINDING AMOUNT OF MI3DNA (ng)
400
300
200
100
0
80
60
40
20
0
BINDING AMOUNT OF MI3DNA / ADDED AMOUNT OF MI3DNA (%)
0
1000
2000
PROBE DNA ON CRYSTAL OSCILLATOR (ng)

FIG. 16

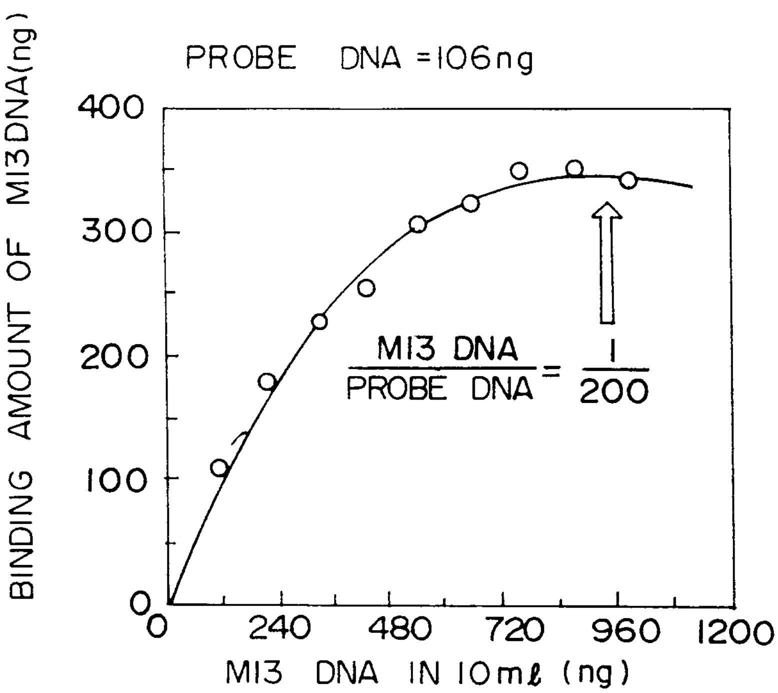
PROBE DNA =106ng
BINDING AMOUNT OF M13DNA(ng)
400
300
200
100
0
0
240
480
720
960
1200
M13 DNA IN 10mℓ (ng)
M13 DNA / PROBE DNA = 1/200

FIG. 17

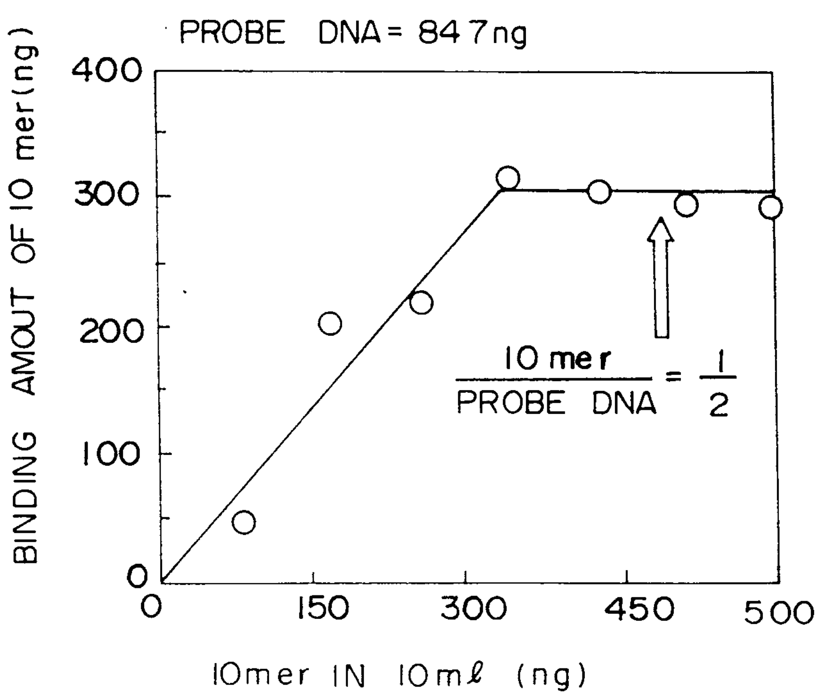
PROBE DNA= 847ng
BINDING AMOUT OF 10 mer(ng)
400
300
200
100
0
0
150
300
450
500
10mer IN 10mℓ (ng)
10 mer / PROBE DNA = 1/2

FIG. 18

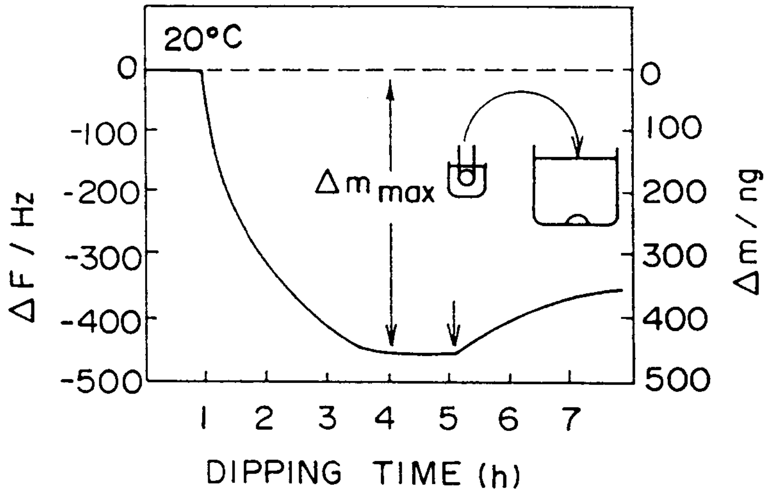
20°C
0
-100
-200
-300
-400
-500
ΔF / Hz
Δm max
0
100
200
300
400
500
Δm / ng
1 2 3 4 5 6 7
DIPPING TIME (h)

FIG. 19

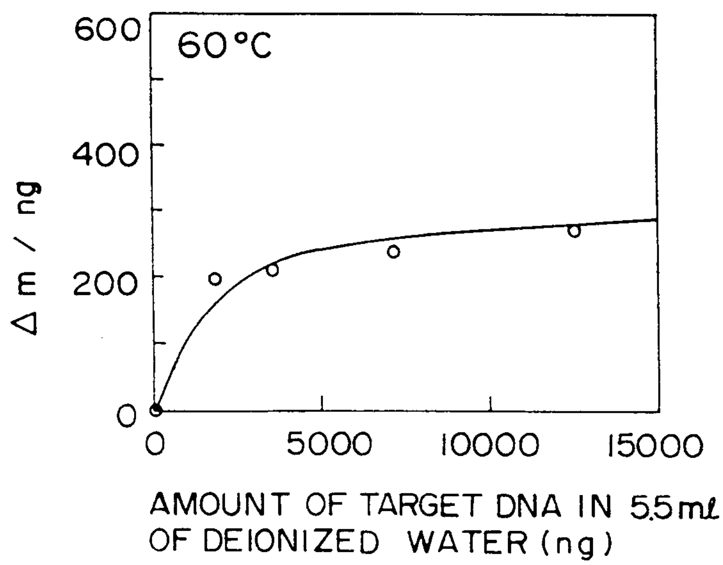
60°C
600
400
200
0
Δm / ng
0 5000 10000 15000
AMOUNT OF TARGET DNA IN 5.5 mℓ
OF DEIONIZED WATER (ng)

FIG. 20

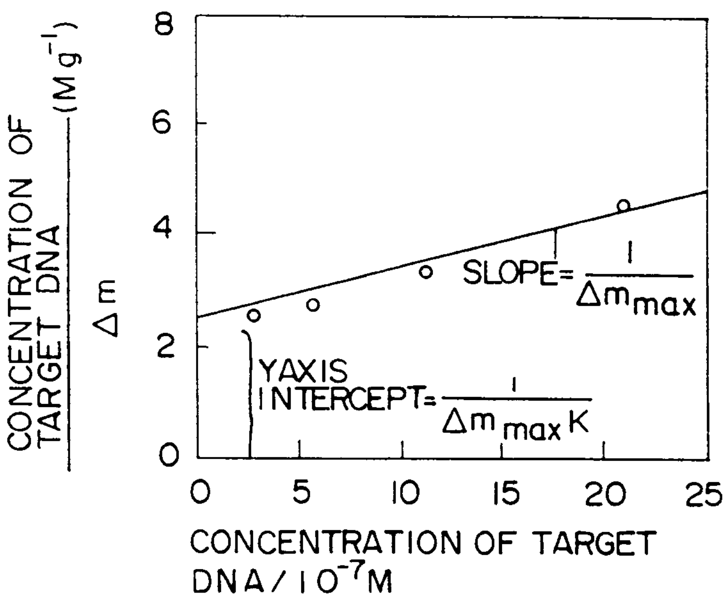
8
6
4
2
0
0
5
10
15
20
25
CONCENTRATION OF TARGET DNA / Δm (Mg⁻¹)
SLOPE= 1/Δm max
YAXIS INTERCEPT= 1/Δm max K
CONCENTRATION OF TARGET DNA/10⁻⁷M